# EUROPÄISCHE PATENTANMELDUNG

(11) **EP 2 273 856 A2**
(43) Veröffentlichungstag der Anmeldung: **12.01.2011**
(21) Anmeldenummer: 10163085.3
(22) Anmeldetag: 18.05.2010
(51) Int. Cl.: H05H 7/18

(54) **Beschleunigeranlage und Verfahren zur Einstellung einer Partikelenergie**

(30) Priorität: 08.07.2009 DE 102009032275
(71) Anmelder: Siemens Aktiengesellschaft, 80333 München (DE)
(72) Erfinder: Bönig, Marc-Oliver, 90419 Nürnberg (DE)

(57) **Zusammenfassung**

Die Erfindung betrifft eine Beschleunigeranlage mit einer Beschleunigereinheit zur Beschleunigung von Partikeln und einer Strahltransportstrecke mit welcher die Partikel von der Beschleunigereinheit an einen von der Beschleunigereinheit entfernten Ort führbar sind, wobei entlang der Strahltransportstrecke eine HF-Kavität angeordnet ist, mit der ein elektromagnetisches HF-Feld erzeugbar ist, das mit den in der Strahltransportstrecke geführten Partikeln wechselwirkt, wobei eine Phase und eine Frequenz des HF-Feldes derart einstellbar sind, dass eine Variation der Energie der mit dem HF-Feld wechselwirkenden Partikeln erzeugbar ist. Weiterhin betrifft die Erfindung ein Verfahren zur Einstellung einer Energie von Partikeln bei welchem die Partikel mit einer Beschleunigereinheit auf ein erstes Energieniveau beschleunigt werden, die beschleunigten Partikel von der Beschleunigereinheit zu einem Bestrahlungsraum geführt werden, wobei die Partikel entlang der Strecke von der Beschleunigereinheit zu dem Bestrahlungsraum durch eine HF-Kavität geführt werden, in der ein HF-Feld auf die Partikel wirkt, wobei eine Phase und eine Frequenz des HF-Feldes derart eingestellt werden, dass die Energie der die HF-Kavität passierenden Partikel verändert wird.

## Beschreibung

Die Erfindung betrifft eine Beschleunigeranlage, mit welcher geladene Teilchen beschleunigt werden und welche insbesondere Einsatz in der Partikeltherapie findet. Weiterhin betrifft die Erfindung ein Verfahren, wie bei einer derartigen Anlage die Energie der Partikel eingestellt werden kann.

Die Partikeltherapie ist ein etabliertes Verfahren zur Behandlung von Gewebe, insbesondere von Tumorerkrankungen. Bestrahlungsverfahren, wie sie in der Partikeltherapie eingesetzt werden, finden jedoch auch in nicht-therapeutischen Gebieten Anwendung. Hierzu gehören beispielsweise Forschungsarbeiten im Rahmen der Partikeltherapie, die an nicht-lebenden Phantomen oder Körpern durchgeführt werden, Bestrahlungen von Materialien, etc. Hierbei werden geladene Partikel wie z.B. Protonen oder Kohlenstoffionen oder andere Ionensorten auf hohe Energien beschleunigt, zu einem Partikelstrahl geformt und über ein Hochenergiestrahltransportsystem zu einem oder mehreren Bestrahlungsräumen geführt. In einem dieser Bestrahlungsräume wird das zu bestrahlende Objekt mit dem Partikelstrahl bestrahlt.

Bei der Bestrahlung eines Zielvolumens wird die Eindringtiefe der Partikel bzw. des Partikelstrahls in das Zielvolumen durch die Energie bestimmt, die die Partikel besitzen. Ein Beschleuniger, z.B. ein Synchrotron oder ein Zyklotron, erzeugt dabei einen im Wesentlichen monoenergetischen Partikelstrahl. Der Partikelstrahls wir auf ein Zielvolumen gelenkt und die quasi monoenergetischen Partikel deponieren ihre Energie innerhalb eines sehr kleinen örtlichen Bereichs entlang der Strahlrichtung, innerhalb des sogenannten Bragg-Peaks.

Es kann vorkommen, dass sich das Zielvolumen bewegt, wobei die Bewegung z.B. durch Atmung, Herzschlag, Darmperistaltik hervorgerufen oder bei einer Bestrahlung von Phantomen gezielt induziert werden kann. Aufgrund derartiger Bewegungen kann es vorkommen, dass die Eindringtiefe der Partikel nicht mehr mit dem gewünschten Ort der Interaktion der Partikel mit dem Zielvolumen übereinstimmt.

Es ist bekannt, die Energie der Partikel nach der Beschleunigung mittel Hilfe eines Keilsystems, z.B. aus Polymethylmethacrylat) zu variieren. In diesem Keilsystem verliert der Teilchenstrahl ja nach Ort, wo der Strahl den Keil durchdringt, entsprechend Energie, so dass die Eindringtiefe verringert wird. Der Keil wird je nach gewünschter Eindringtiefe entsprechend in den Strahl gefahren. Eine derartige Vorrichtung ist z.B. aus der US 6,710,362 B2 bekannt.

Die WO 2009/026997 A1 offenbart ein anderes System zur Variation der Energie des Partikelstrahls mit einem ortsfesten Keil.

Es ist die Aufgabe der Erfindung, eine Beschleunigeranlage bereitzustellen, mit welcher eine schnelle und genaue Einstellung der Energie des Partikelstrahls bei gleichzeitig hoher Strahlqualität möglich ist. Weiterhin ist es die Aufgabe der Erfindung, ein Verfahren zum Einstellen einer Energie des Partikelstrahls bereitzustellen, das es erlaubt, die Energie bei hoher Strahlqualität schnell und genau einzustellen.

Die Aufgabe der Erfindung wird gelöst durch die Merkmale der unabhängigen Ansprüche. Vorteilhafte Weiterbildungen der Erfindung finden sich in den Merkmalen der abhängigen Ansprüche.

Die vorstehenden und die nachfolgenden Ausführungen zu Merkmalen, deren Wirkungsweise und deren Vorteile beziehen sich jeweils auf die Vorrichtungskategorie und auf die Verfahrenskategorie, ohne dass dies jeweils explizit erwähnt ist. Die dabei offenbarten Einzelmerkmale können auch in anderen als den gezeigten Kombinationen erfindungswesentlich sein.

Die erfindungsgemäße Beschleunigeranlage weist auf:
- eine Beschleunigereinheit zur Beschleunigung von Partikeln auf z.B. eine zur Bestrahlung eines Zielvolumens notwendige Energie,
- eine Strahltransportstrecke, welche an die Beschleunigereinheit anschließt und mit welcher die Partikel, die mit der Beschleunigereinheit beschleunigt und aus der Beschleunigereinheit ausgeleitet worden sind, von der Beschleunigereinheit an einen von der Beschleunigereinheit entfernten Ort, z.B. einem Bestrahlungsraum, führbar sind,
wobei entlang der Strahltransportstrecke eine HF-Kavität angeordnet ist, mit der ein elektromagnetisches HF-Feld erzeugbar ist, das mit den in der Strahltransportstrecke geführten Partikeln wechselwirkt, wobei eine Phase und eine Frequenz des HF-Feldes derart einstellbar sind, dass eine Variation der Energie der mit dem HF-Feld wechselwirkenden Partikeln erzeugbar ist.

Die Beschleunigereinheit ist dabei derart ausgebildet, dass mit der Beschleunigereinheit eine Beschleunigung der Partikel auf mindestens ein Energieniveau erzeugt werden kann, welches z.B. einer Eindringtiefe in einen wasseräquivalenten Körper von mindestens 15 cm entspricht, insbesondere von mindestens 20 cm und höchst insbesondere von mindestens 25cm. Mit der Beschleunigereinheit können Partikel z.B. auf die über 50 MeV beschleunigt werden. Typische bei einer Bestrahlung verwendete Energien liegen bei Protonen im Bereich von 48 MeV/u bis 250 MeV/u und mehr und im Bereich von Kohlenstoffionen im Bereich von 85 MeV/u bis 430 MeV/u und mehr. Die Beschleunigereinheit kann hierzu einen Kreisbeschleuniger umfassen, wie beispielsweise ein Synchrotron oder ein Zyklotron.

Die Partikel werden aus der Beschleunigereinheit ausgeleitet oder extrahiert und anschließend zu einem Bestrahlungsraum geführt. Dies erfolgt über ein Strahltransportsystem, das üblicherweise eine Vakuumsröhre und eine Vielzahl von Dipol- und Quadrupolmagneten zum Umlenken des Strahls und zur Fokussierung bzw. Defokussierung aufweist. In dem Strahltransportsystem findet bei herkömmlichen Beschleunigeranlagen üblicherweise keine weitere Energieänderung der Partikel statt.

Entlang dieses Strahltransportsystems befindet sich bei Ausführungsformen der vorliegenden Erfindung eine zusätzliche HF-Kavität, mit der eine weitere Beschleunigung bzw. eine Entschleunigung der die HF-Kavität durchquerenden Partikel erreicht werden kann. Dies geschieht über das von der HF-Kavität erzeugte elektromagnetische HF-Feld, welches auf die Partikel eingestrahlt wird. Die Frequenz des elektromagnetischen HF-Feldes ist dabei auf die Bunchfrequenz der Partikel abgestimmt.

Weiterhin ist das elektromagnetische HF-Feld, das von der HF-Kavität auf die Partikel eingestrahlt wird, auf die Zeitpunkte abgestimmt, an denen jeweils ein Partikelpaket (Bunch) die Kavität durchquert. Die Phase des HF-Feldes kann dabei derart auf die jeweils die Kavität durchquerenden Partikelpakete abgestimmt sein, dass die Partikelpakete je nach Einstellung beschleunigt, abgebremst oder nicht beeinflusst werden.

Insbesondere kann die Beschleunigeranlage als Partikeltherapieanlage ausgebildet sein, wobei die Beschleunigeranlage eine Steuerungsvorrichtung aufweist, welche ausgebildet ist zum Laden eines Bestrahlungsplanungsdatensatzes und zur Steuerung der Beschleunigeranlage in Abhängigkeit von dem geladenen Bestrahlungsplanungsdatensatz. Der Bestrahlungsplanungsdatensatz umfasst die Steuerparameter, die eine Bestrahlung des Zielvolumens gemäß bestimmter Vorgaben, die zuvor festgelegt wurden, erlauben.

Insbesondere kann der Bestrahlungsplanungsdatensatz zumindest einen Parameter umfassen, welche eine einzustellende Partikelenergie charakterisiert. In diesem Fall ist die Steuerungsvorrichtung derart ausgebildet, dass die Partikelenergie durch eine Kombination der Ansteuerung der Beschleunigereinheit und der HF-Kavität eingestellt werden kann. Die einzustellende Partikelenergie kann beispielsweise erzeugt werden, indem die Beschleunigereinheit die Partikel auf ein erstes Energieniveau beschleunigt, welche von der Partikelenergie abweichen kann, und dass anschließend die Differenz zwischen dem ersten Energieniveau und der einzustellenden Partikelenergie durch die HF-Kavität ausgeglichen wird.

Auf diese Weise kann beispielsweise die Energie des Partikelstrahls schnell und einfach eingestellt werden. Wenn beispielsweise bei einer schichtweisen Bestrahlung die Energie des Partikelstrahls geändert wird, um den Partikelstrahl von einer Schicht auf die nächste Schicht einzustellen, muss die Energie des Partikelstrahls geändert werden. Wenn die Energie des Partikelstrahls jeweils in der Beschleunigereinheit eingestellt werden muss, ist dies vergleichsweise zeitaufwändig, da beispielsweise bei einem Synchrotron alle Magnete neu eingestellt und kontrolliert werden müssen.

Mithilfe der HF-Kavität gelingt es jedoch schnell und einfach die Energie des Partikelstrahls in einem gewissen Umfang zu variieren, ohne die Beschleunigereinheit hierfür verändern zu müssen. Die Ansteuerung der HF-Kavität - und damit die Änderung der Energie des Partikelstrahls - lässt sich vergleichsweise sehr schnell durchführen. Lediglich wenn die Energie des Partikelstrahls in einem Ausmaß geändert werden soll, das die Möglichkeit der HF-Kavität übersteigt, ist eine Änderung der Einstellung der Beschleunigereinheit notwendig.

In einer vorteilhaften Ausführungsform umfasst die Beschleunigeranlage eine Vorrichtung, der die Position eines zu bestrahlenden Zielvolumens erfasst werden kann. Beispielsweise kann die Beschleunigeranlage eine Schnittstelle aufweisen, mit der Signale eines Atemsensors registriert werden können. Die Signale des Atemsensors erlauben dabei einen Rückschluss auf die Atembewegung. Hieraus lässt sich die Position eines Zielvolumens, dass durch die Atembewegung verschoben wird, ermitteln. Die Vorrichtung, mit der die Position des zu bestrahlenden Zielvolumens erfasst werden kann, kann folglich auch ein Signal erfassen, das einen indirekten Rückschluss auf die Position des Zielvolumens erlaubt. Der Atemsensor ist dabei nur ein beispielhaftes Mittel, es können z.B. ebenso Röntgenvorrichtungen eingesetzt werden, mit denen sich die Position des Zielvolumens überwachen lässt, oder andere Vorrichtungen, wie sie für die Positionsüberwachung eines Zielvolumens bekannt sind.

Die Steuerungsvorrichtung kann dann die Energie der Partikel in Abhängigkeit der Position des zu bestrahlenden Zielvolumens durch Ansteuerung der HF-Kavität entsprechend variieren, um z.B. die Energie der Teilchen an das zu durchquerende Gewebe, das in Strahlrichtung vor dem Zielvolumen liegt, schnell anzupassen. Diese Variation ist möglich, da die Ansteuerung der HF-Kavität sehr schnell ist, so dass der Partikelstrahl der Bewegung des Zielvolumens nachgeführt werden kann. Dabei kann die Phase des HF-Feldes vorzugsweise stufenlos variiert werden, um eine stufenlose Änderung der Eindringtiefe des Partikelstrahls zu erreichen.

Im Vergleich zu Tiefenmodulationsvorrichtungen, welche ein in den Partikelstrahl einbringbares Material aufweisen, hat diese Ausführungsform den Vorteil, dass die Qualität des Partikelstrahls nicht durch Material beeinträchtigt wird, durch das der Partikelstrahl geführt wird. Es wird die Spallation des Primärstrahls in Materie vermieden, wodurch der Patient einer geringeren Strahlenbelastung ausgesetzt wird. Insbesondere kann so Gewebe geschont werden, das nicht mit Strahlung belastet werden soll. Der Partikelstrahl weitet sich weniger auf, so dass sich ein insgesamt kleinerer Strahlfleck im Isozentrum und damit eine bessere Strahlqualität und eine genauere Bestrahlung des Zielvolumens erreichen lässt.

Die HF-Kavität ist bevorzugt supraleitend ausgebildet, um weniger Platz einzunehmen.

Die HF-Kavität ist insbesondere derart dimensioniert, dass mit HF-Kavität eine Energiemodulation des in HF-Kavität eintretenden Partikelstrahls erreicht wird, welche einer Modulation der Eindringtiefe der Partikel in einem wasseräquivalenten Körper von mindestens 1 cm, insbesondere von mindestens 2 cm und höchst insbesondere von mindestens 3 cm oder sogar mehr entspricht. Auf diese Weise können bei einer Partikeltherapieanlage typische Bewegungen eines Zielvolumens abgebildet werden.

Dies kann erreicht werden, indem die HF-Kavität derart beschaffen ist, dass das erzeugbare HF-Feld eine maximale Feldstärke von mindestens 20 MV/m beträgt. Bevorzugterweise beträgt die maximale Feldstärke mindestens 40 MV/m oder 50 MV/m. Hierdurch lassen sich Variationen der Strahlenergie erreichen, welche bis zu 50 MeV betragen und damit bei Protonen z.B. eine Änderung der Eindringtiefe von 2 cm bis 3 cm Wasseräquivalenz erzeugen. Derartige Feldstärken und Änderungen der Energie der Partikel können dann mit einer HF-Kavität erreicht werden, deren Länge 1 m bis 2 m beträgt. Derartig dimensionierte HF-Kavitäten lassen sich in einer Strahltransportstrecke gut einbauen, ohne eine herkömmliche Strahltransportstrecke stark umbauen oder ändern zu müssen.

Bei dem erfindungsgemäßen Verfahren zur Einstellung einer Energie von Partikeln, welche in einer Beschleunigeranlage beschleunigt werden, werden
- die Partikel mit einer Beschleunigereinheit auf ein erstes Energieniveau beschleunigt,
- die beschleunigten Partikel von der Beschleunigereinheit zu einem Bestrahlungsraum geführt,
wobei die Partikel entlang der Strecke von der Beschleunigereinheit zu dem Bestrahlungsraum durch eine HF-Kavität geführt werden, in der ein HF-Feld auf die Partikel wirkt, wobei eine Phase und eine Frequenz des HF-Feldes derart gesteuert werden, dass die Energie der die HF-Kavität passierenden Partikel verändert wird.

Die Kombination aus Beschleunigung auf eine erste Energiestufe und aus nachfolgender Veränderung der Energie kann derart gesteuert werden, dass die Partikel nach Verlassen der HF-Kavität eine vordefinierte, z.B. in einem Bestrahlungsplanungsdatensatz hinterlegte Energie aufweisen.

Die Energie der auf das erste Energieniveau beschleunigten Partikel kann z.B. durch insbesondere stufenlose Variation der Phase des auf die Partikel wirkenden Feldes variabel verändert werden.

Das Verfahren kann vor allem dazu eingesetzt werden, die auf das erste Energieniveau beschleunigten Partikel in Abhängigkeit einer Bewegung eines zu bestrahlenden Zielvolumens zu verändern. Hierdurch kann der Partikelstrahl einer Bewegung des Zielvolumens nachgeführt werden.

Ausführungsformen der Erfindung mit vorteilhaften Weiterbildungen gemäß den Merkmalen der abhängigen Ansprüche werden anhand der folgenden Zeichnung näher erläutert, ohne jedoch darauf beschränkt zu sein. Es zeigen:
- Fig. 1: einen schematischen Überblick über den Aufbau einer Partikeltherapieanlage,
- Fig. 2: ein Diagramm zur Erläuterung der Wechselwirkung des durch die HF-Kavität erzeugten HF-Feldes mit Teilchen- paketen, und
- Fig. 3: eine Abfolge von Schritten, die bei der Durchführung des Verfahrens ausgeführt werden können.

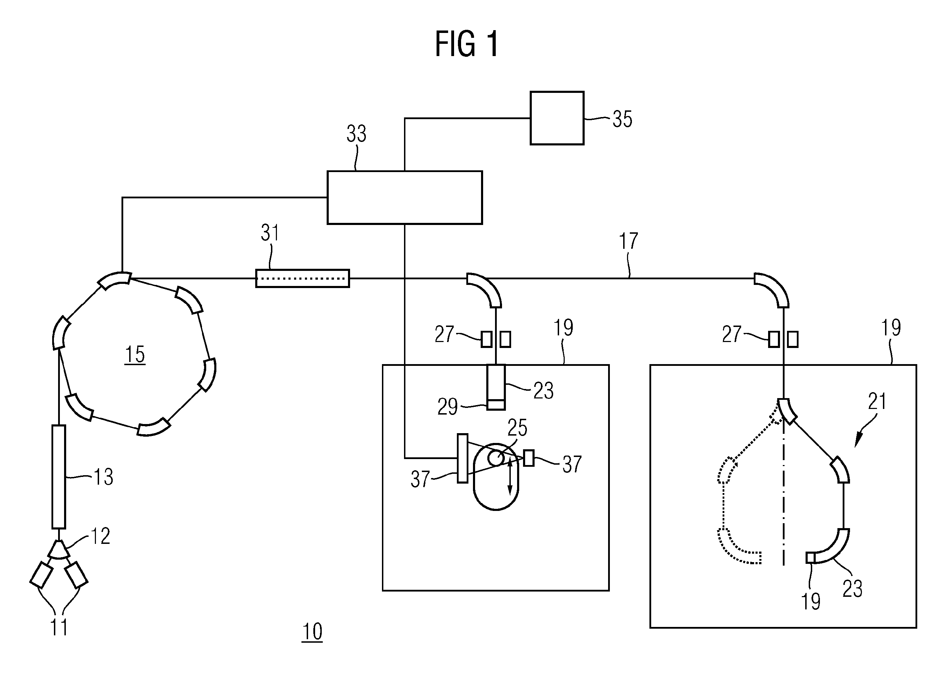

Figur 1 zeigt einen schematischen, nicht maßstabsgetreuen Überblick über den Aufbau einer Partikeltherapieanlage 10. In einer Partikeltherapieanlage 10 erfolgt insbesondere eine Bestrahlung eines Körpers, insbesondere eines tumorerkrankten Gewebes, mit einem Partikelstrahl. Es können jedoch auch Phantome oder Zellkulturen beispielsweise zu Forschungszwecken oder zu Wartungszwecken bestrahlt werden.

Als Partikel werden vornehmlich Ionen wie beispielsweise Protonen, Pionen, Heliumionen, Kohlenstoffionen oder andere Ionensorten eingesetzt. Üblicherweise werden derartige Partikel in einer Partikelquelle 11 erzeugt. Wenn, wie in Figur 1 dargestellt, zwei Partikelquellen 11 vorhanden sind, die zwei verschiedene Ionensorten erzeugen, kann zwischen diesen beiden Ionensorten innerhalb eines kurzen Zeitintervalls umgeschaltet werden. Dazu wird beispielsweise ein Schaltmagnet 12 verwendet, der zwischen den Ionenquellen 11 einerseits und einem Vorbeschleuniger 13 andererseits angeordnet ist. Z.B. kann hierdurch die Partikeltherapieanlage 10 mit Protonen und mit Kohlenstoffionen gleichzeitig betrieben werden.

Die von der oder einer der Ionenquellen 11 erzeugten und gegebenenfalls mit dem Schaltmagneten 12 ausgewählten Ionen werden in dem Vorbeschleuniger 13 auf ein erstes Energieniveau beschleunigt. Der Vorbeschleuniger 13 ist beispielsweise ein Linearbeschleuniger (LINAC für engl.: "LINear ACcelerator"). Anschließend werden die Partikel in einen Beschleuniger 15, beispielsweise ein Synchrotron oder Zyklotron, eingespeist. In dem Beschleuniger 15 werden sie auf hohe Energien, wie sie zur Bestrahlung nötig sind, beschleunigt.

Nachdem die Partikel den Beschleuniger 15 verlassen, führt ein Hochenergiestrahl-Transportsystem 17 den Partikelstrahl zu einem oder mehreren Bestrahlungsräumen 19. In einem Bestrahlungsraum 19 werden die beschleunigten Partikel auf einen zu bestrahlenden Körper gerichtet. Je nach Ausgestaltung erfolgt dies von einer festen Richtung (in so genannten "fixed beam"-Räumen) aus oder aber über eine um eine Achse bewegliche rotierbare Gantry 21 von verschiedenen Richtungen aus.

Im Bestrahlungsraum 19 tritt der Partikelstrahl aus einem Strahlauslass 23 aus und trifft auf ein zu bestrahlendes

Zielvolumen 25, das sich üblicherweise im Isozentrum eines Bestrahlungsraums 19 befindet.

Die Partikeltherapieanlage 10 kann weiterhin ein System von Scanmagneten 27 aufweisen, mit denen der Partikelstrahl abgelenkt und über das Zielvolumen 25 gescannt werden kann, sowie einen Monitorsystem 29, mit dem verschiedene Partikelstrahlparameter überwacht werden können.

In das Hochenergiestrahl-Transportsystem 17 ist eine HF-Kavität 31 integriert. Die HF-Kavität 31 ermöglicht es, ein HF-Feld auf den Partikelstrahl einwirken zu lassen, wenn Partikelpakete (so genannte "Bunches") des Partikelstrahls die HF-Kavität 31 durchqueren. Vom Prinzip und von der Funktionsweise her gleicht die HF-Kavität 31 einer HF-Kavität, wie sie in einem Synchrotron zur Beschleunigung von den im Synchrotron zirkulierenden Partikelpaketen eingesetzt wird.

In Fig. 1 ist eine HF-Kavität 31 gezeigt, welche in der Strahltransportstrecke vor den Umlenkmagneten angeordnet ist, mit denen der Partikelstrahl zu den einzelnen Bestrahlungsräumen 19 umgelenkt wird. Dies hat zwar den Vorteil, dass die HF-Kavität 31 von allen Bestrahlungsräumen 19 gemeinsam genutzt werden kann, sodass die Anlage kostengünstig wird. Nachteilig bei einer derartigen Ausgestaltung ist, dass auch das Magnetfeld der nachfolgenden Umlenkmagnete der durch die HF-Kavität 31 erzeugten Energieänderung angepasst werden muss. Dies kann gegebenenfalls die Geschwindigkeit begrenzen, mit der eine Energieänderung gesteuert oder geregelt werden kann.

In einer anderen (hier die Übersichtlichkeit halber nicht gezeigten) Ausführungsform kann die HF-Kavität 31 auch entlang der Strahltransportstrecke nach dem Umlenkmagneten angeordnet werden, der den Partikelstrahl in einen der Bestrahlungsräume 19 lenkt. Hierdurch ist es möglich, eine schnellere Variation der Energie der Partikel mit der HF-Kavität 31 zu erzeugen, da weniger oder gar keine nachfolgenden Magnete an die durch die HF-Kavität 31 erzeugte Energieänderung angepasst werden müssen. Dies ist insbesondere beim Tracking einer Bewegung des Zielvolumens 25 vorteilhaft. Allerdings muss dann eine derartige HF-Kavität 31 für jeden Bestrahlungsraum 19 vorgesehen werden, für welchen eine Energieänderung der Partikel mittels einer HF-Kavität 31 ermöglicht werden soll.

Die Frequenz, mit der derartige Partikelpakete die HF-Kavität 31 durchquert, hängt unter anderem von dem Energieniveau ab, mit der die Partikel durch den Beschleuniger 15 beschleunigt werden. Die Frequenz des HF-Feldes ist auf die Frequenz der Partikelpakete abgestimmt.

Weiterhin ist die Phase des HF-Feldes derart auf die Zeitpunkte, an denen die Partikelpakete die HF-Kavität 31 durchqueren, abgestimmt, dass die Energie der Partikelpakete entweder zusätzlich erhöht, erniedrigt oder gleich belassen wird.

Um dies zu erreichen, weist die Partikeltherapieanlage 10 eine Steuerungsvorrichtung 33 auf, in welche z.B. ein Bestrahlungsplanungsdatensatz 35 geladen werden kann, um die Partikeltherapieanlage 10 zu steuern, um den zugehörigen Bestrahlungsplan umzusetzen. Die Steuerungsvorrichtung 33 steuert die Komponenten der Partikeltherapieanlage 10 entsprechend, d.h. auch den Beschleuniger 15 und die HF-Kavität 31, und steht demzufolge mit den zu steuernden Komponenten in Verbindung (der Übersichtlichkeit halber sind nur einige wenige Verbindungen gezeigt).

Im Bestrahlungsraum 19 kann zusätzlich eine Bewegungsüberwachungsvorrichtung 37 vorgesehen sein, um die Bewegung des Zielvolumens 25 verfolgen zu können, z.B. eine Fluoroskopievorrichtung. Die von der Bewegungsüberwachungsvorrichtung 37 aufgezeichneten Daten werden über eine Schnittstelle der Steuerungsvorrichtung 33 übermittelt, welche darauf basierend die Energievariation bestimmt, die notwendig ist, um den Partikelstrahl der Bewegung des Zielvolumens 25 nachführen zu können. Die HF-Kavität 31 wird entsprechend gesteuert.

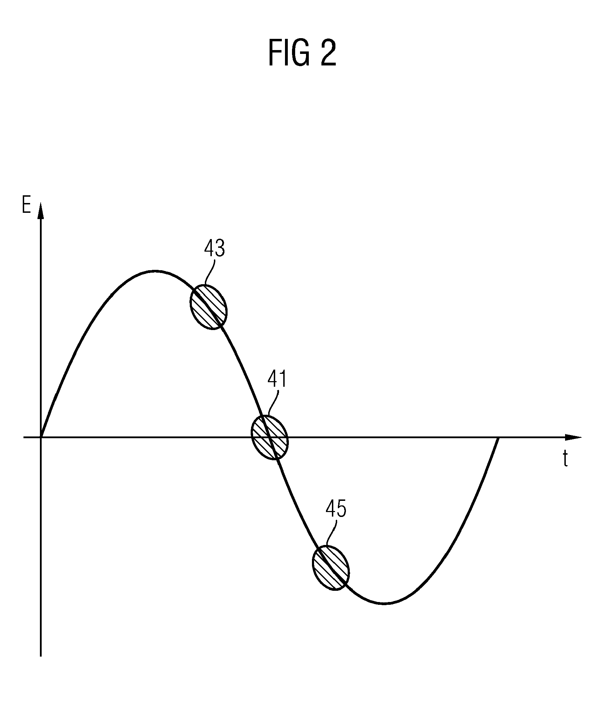

Fig. 2 zeigt anhand eines Diagramms die Abstimmung der Phase des HF-Feldes mit den Partikelpaketen, auf die des HF-Feld einwirkt.

Das Diagramm zeigt die zeitliche Änderung des von der HF-Kavität eingestrahlten elektrischen Feldes E. befindet sich das Feld an dem Zeitpunkt, an dem ein Partikelpaket durch die HF-Kavität tritt, am Nulldurchgang, wird die Energie des Partikelpakets nicht geändert (Punkt 41). Ist die Phase des Feldes jedoch in eine Richtung verschoben (Punkt 43), findet eine Beschleunigung des Partikelpaket statt. Ist die Phase in die andere Richtung verschoben (Punkt 45) wird das Partikelpaket abgebremst. Um zwischen den einzelnen Punkten hin und her zu wechseln, kann die Phase zwischen den Partikelpaketen und der HF-Welle stufenlos verschoben werden. So wird eine stufenlose Variation der Strahlenergie in einem gewissen Umfang erreicht.

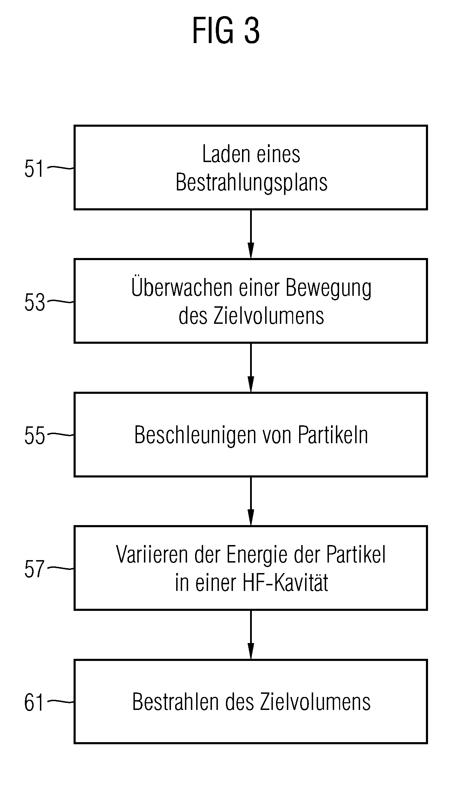

Fig. 3 zeigt Verfahrensschritte, die bei einer Ausführungsform des Verfahrens ausgeführt werden.

In einem ersten Schritt wird ein Bestrahlungsplanungsdatensatz in die Steuerungsvorrichtung eine Partikeltherapieanlage geladen (Schritt 51). In dem Bestrahlungsplanungsdatensatz sind Daten einer Bestrahlungsplanung hinterlegt, welche angeben, wie eine Bestrahlung des Zielvolumens stattzufinden hat, um eine gewünschte Solldosisverteilung im Zielvolumen zu deponieren.

Anschließend wird begonnen, die Bewegung des Zielvolumens zu überwachen und zu erfassen (Schritt 53).

Es wird ein Partikelstrahl erzeugt, der geeignet ist, den Bestrahlungsplanungsdatensatz umzusetzen. Hierzu werden die Partikel zunächst in dem Beschleuniger auf eine erste Energiestufe beschleunigt (Schritt 55). Anschließend wird die Energie wird der Partikel mithilfe der HF-Kavität variiert (Schritt 57). Das Steuern der Beschleunigereinheit und der HF-Kavität erfolgt gemäß der Vorgabe, die Bestrahlungsplanungsdatensatz hinterlegt ist und der erfassten Bewegungsposition des Zielvolumens.

Mit dem Partikelstrahl, dessen Energie mithilfe der Beschleunigereinheit und der HF-Kavität eingestellt wurde, wird das Zielvolumen bestrahlt (Schritt 59).

### Bezugszeichenliste

- 10: Partikeltherapieanlage
- 11: Partikelquelle
- 12: Schaltmagnet
- 13: Vorbeschleuniger
- 15: Beschleuniger
- 17: Hochenergiestrahl-Transportsystem
- 19: Bestrahlungsraum
- 21: Gantry
- 23: Strahlauslass
- 25: Zielvolumen
- 27: Scanmagnete
- 29: Monitorsystem
- 31: HF-Kavität
- 33: Steuerungsvorrichtung
- 35: Bestrahlungsplanungsdatensatz
- 37: Bewegungsüberwachungsvorrichtung

- 4x: Punkt 4x

- 5x: Schritt 5x

## Patentansprüche

1. Beschleunigeranlage, aufweisend
- eine Beschleunigereinheit (13, 15) zur Beschleunigung von Partikeln,
- eine Strahltransportstrecke (17), welche an die Beschleunigereinheit (13, 15) anschließt und mit welcher die Partikel, die mit der Beschleunigereinheit (13, 15) beschleunigt und aus der Beschleunigereinheit (13, 15) ausgeleitet worden sind, von der Beschleunigereinheit (13, 15) an einen von der Beschleunigereinheit (13, 15) entfernten Ort (19) führbar sind,
**dadurch gekennzeichnet, dass**
entlang der Strahltransportstrecke (17) eine HF-Kavität (31) angeordnet ist, mit der ein elektromagnetisches HF-Feld (E) erzeugbar ist, das mit den in der Strahltransportstrecke (17) geführten Partikeln wechselwirkt,
wobei eine Phase und eine Frequenz des HF-Feldes (E) derart einstellbar sind, dass eine Variation der Energie der mit dem HF-Feld (E) wechselwirkenden Partikeln erzeugbar ist.

2. Beschleunigeranlage nach Anspruch 1, wobei die Beschleunigeranlage als Partikeltherapieanlage (10) ausgebildet ist, wobei die Beschleunigeranlage eine Steuerungsvorrichtung (33) aufweist, welche ausgebildet ist zum Laden eines Bestrahlungsplanungsdatensatzes (35) und zur Steuerung der Beschleunigeranlage in Abhängigkeit von dem geladenen Bestrahlungsplanungsdatensatz (35).

3. Beschleunigeranlage nach Anspruch 1, wobei der Bestrahlungsplanungsdatensatz (35) zumindest einen Parameter umfasst, welcher eine einzustellende Partikelenergie charakterisiert,
und wobei die Steuerungsvorrichtung (33) ausgebildet ist zur Einstellung der Partikelenergie, indem die Beschleunigereinheit (13, 15) und die HF-Kavität (31) derart angesteuert werden, dass die Beschleunigereinheit (13, 15) die Partikel auf eine erste Energiestufe beschleunigt, welche anschließend durch die HF-Kavität (31) verändert wird, sodass die im Bestrahlungsplanungsdatensatz (35) hinterlegte Partikelenergie eingestellt wird.

4. Beschleunigeranlage nach einem der Ansprüche 2 oder 3, wobei die Beschleunigeranlage eine Vorrichtung (37) zum Erfassen einer Position eines zu bestrahlenden Zielvolumens (25) aufweist, und wobei die Steuerungsvorrichtung (33) derart ausgebildet ist zum Ansteuern der HF-Kavität (31) in Abhängigkeit der von der Position des zu bestrahlenden Zielvolumens (25).

5. Beschleunigeranlage nach einem der Ansprüche 1 bis 4, wobei die HF-Kavität (31) supraleitend ist.

6. Beschleunigeranlage nach einem der Ansprüche 1 bis 5, wobei die HF-Kavität (31) derart dimensioniert ist, dass durch sie ein HF-Feld mit einer Feldstärke von mindestens 20 MV/m erzeugbar ist.

7. Beschleunigeranlage nach einem der Ansprüche 1 bis 6, wobei die HF-Kavität (31) sich über eine Länge in Strahlverlaufsrichtung von über mindestens 1 m erstreckt.

8. Beschleunigeranlage nach einem der Ansprüche 1 bis 7, wobei die HF-Kavität (31) derart dimensioniert ist, dass mit der HF-Kavität (31) eine Energiemodulation des die HF-Kavität (31) durchquerenden Partikelstrahls erreicht wird, welche einer Modulation der Eindringtiefe in einen wasseräquivalenten Körper von mindestens 1 cm entspricht.

9. Beschleunigeranlage nach einem der Ansprüche 1 bis 8, wobei mit der Beschleunigereinheit die Partikel auf eine Energie beschleunigbar sind, welche einer Eindringtiefe in einen wasseräquivalenten Körper von mindestens 15 cm entspricht.

10. Verfahren zur Einstellung einer Energie von Partikeln, welche in einer Beschleunigeranlage beschleunigt werden, bei welchem
- die Partikel mit einer Beschleunigereinheit (13, 15) auf ein erstes Energieniveau beschleunigt werden,
- die beschleunigten Partikel von der Beschleunigereinheit (13, 15) zu einem Bestrahlungsraum (19) geführt werden,
**dadurch gekennzeichnet, dass**
die Partikel entlang der Strecke von der Beschleunigereinheit (13, 15) zu dem Bestrahlungsraum (19) durch eine HF-Kavität (31) geführt werden, in der ein HF-Feld (E) auf die Partikel wirkt, wobei eine Phase und eine Frequenz des HF-Feldes (E) derart eingestellt werden, dass die Energie der die HF-Kavität (31) passierenden Partikel verändert wird.

11. Verfahren nach Anspruch 10, wobei
eine vordefinierte Energie für die Partikel eingestellt wird, indem die Partikel zuerst auf das erste Energieniveau beschleunigt werden und anschließend mit Hilfe der HF-Kavität (31) die Energie der auf das erste Energieniveau beschleunigten Partikel verändert wird, um die zu vordefinierte Energie einzustellen.

12. Verfahren nach Anspruch 10 oder 11, wobei die Energie der auf das erste Energieniveau beschleunigten Partikel durch Variation der Phase des auf die Partikel wirkenden HF-Feldes (E) variabel verändert wird.

13. Verfahren nach einem der Ansprüche 10 bis 12, wobei die Energie der auf das erste Energieniveau beschleunigten Partikel in Abhängigkeit einer Bewegung eines zu bestrahlenden Zielvolumens (35) verändert wird.
